(19) Europäisches Patentamt
European Patent Office
Office européen des brevets

(11) Publication number: 0 294 069 A2

(12) # EUROPEAN PATENT APPLICATION

(21) Application number: 88304661.7

(22) Date of filing: 23.05.88

(51) Int. Cl.[4]: C07D 473/32 , C07D 473/18 , C07D 473/16 , A61K 31/52

Claims for the following Contracting State: ES.

The title of the invention has been amended (Guidelines for Examination in the EPO, A-III, 7.3).

(30) Priority: 30.05.87 GB 8712744

(43) Date of publication of application: 07.12.88 Bulletin 88/49

(84) Designated Contracting States: AT BE CH DE ES FR GB GR IT LI LU NL SE

(71) Applicant: BEECHAM GROUP PLC
Beecham House Great West Road
Brentford Middlesex TW8 9BD(GB)

(72) Inventor: Harnden, Michael Raymond
Beecham Pharmaceuticals
Great Burgh Yew Tree Bottom Road
Epsom Surrey, KT18 5XQ(GB)
Inventor: Wyatt, Paul Graham Beecham Pharmaceuticals
Great Burgh Yew Tree Bottom Road
Epsom Surrey, KT18 5XQ(GB)
Inventor: Bailey, Stuart Beecham Pharmaceuticals
Great Burgh Yew Tree Bottom Road
Epsom Surrey, KT18 5XQ(GB)

(74) Representative: Jones, Pauline et al
Beecham Pharmaceuticals Patents & Trade Marks Dept. Great Burgh Yew Tree Bottom Road
Epsom Surrey KT18 5XQ(GB)

(54) **Purine compounds, process for their preparation, intermediates and pharmaceutical compositions.**

(57) The (S)-isomer of a compound of formula (I), optionally in admixture with up to 45% of the mixture by weight of the corresponding (R)-isomer:

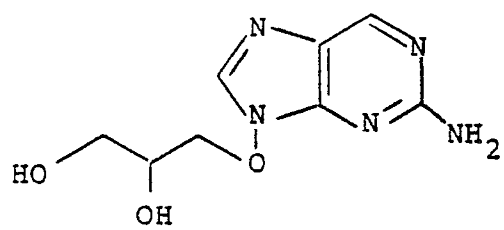

(I)

wherein the OH groups in the chain are optionally in the form of O-acyl, phosphate, cyclic acetal or cyclic carbonate derivatives thereof; having antiviral activity, a process for preparation and use as pharmaceuticals.

EP 0 294 069 A2

## ACTIVE COMPOUNDS

The present invention relates to one isomeric form of chemical compounds having antiviral activity, to processes for their preparation of such isomers and to their use as pharmaceuticals.

EP-A-242482, the subject matter of which is incorporated herein by reference, discloses antiviral compounds of formula (A) and pharmaceutically acceptable salts thereof:

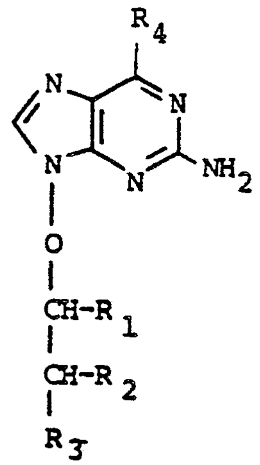

(A)

wherein

$R_1$ is hydrogen or $CH_2OH$;

$R_2$ is hydrogen or, (when $R_1$ is hydrogen), hydroxy or $CH_2OH$;

$R_3$ is $CH_2OH$ or, (when $R_1$ and $R_2$ are both hydrogen), $CH(OH)CH_2OH$;

$R_4$ is hydrogen, hydroxy, amino or $OR_5$

wherein

$R_5$ is $C_{1-6}$ alkyl, phenyl or phenyl $C_{1-2}$ alkyl either of which phenyl moieties may be substituted by one or two halo, $C_{1-4}$ alkyl or $C_{1-4}$ alkoxy groups;

and in which any OH groups in $R_1$, $R_2$ and/or $R_3$ may be in the form of O-acyl, phosphate, cyclic acetal or cyclic carbonate derivatives thereof.

Example 7 describes the (R,S) racemic mixture of the compound, 2-amino-9-(2,3-dihydroxyprop-1-oxy)-purine, and Example 30 describes the pure (R)-isomer prepared by stereospecific synthesis. The pure (S)-isomer has now been prepared and has been found to have greater potency as an antiviral agent as compared with the (R)-isomer. The compounds of formula (A) wherein $R_4$ is H are believed to be pro-drugs for the compounds of formula (A) wherein $R_4$ is OH. Example 31 of EP-A-242482 describes (S)-9-(2,3-dihydroxyprop-1-oxy)guanine.

Accordingly, the present invention provides the (S)-isomer of a compound of formula (I), optionally in admixture with up to 45% of the mixture by weight of the corresponding (R)-isomer:

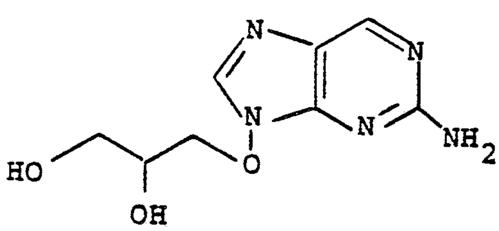

(I)

wherein the OH groups in the chain are optionally in the form of O-acyl, phosphate, cyclic acetal or cyclic carbonate derivatives thereof.

O-Acyl derivatives are normally those wherein one or both of the OH groups in the side chain form carboxylic ester groups; such as $C_{1-7}$ alkanoyl and benzoyl optionally substituted by one or two $C_{1-4}$ alkyl, $C_{1-4}$ alkoxy, halogen or $CF_3$ groups. Preferably, carboxylic ester groups are $C_{1-7}$ alkanoyl groups, such as acetyl, propionyl, butyryl, heptanoyl and hexanoyl, most preferably acetyl or propionyl.

Examples of phosphate esters of the compounds of formula (I) include those where one of the acyclic -OH groups is replaced by $(HO)_2$-$PO_2$- groups or salts thereof, or where the two -OH groups are replaced by a bridging -O-P(OH)$O_2$- group.

The two acyclic OH groups may also be replaced by a group, cyclic acetal group, such as -O-C-($C_{1-3}$alkyl)$_2$-O- or cyclic carbonate, such as -O-CO-O-.

Preferably, the (S)-isomer of a compound of formula (I) or a derivative thereof as defined is in a form containing from 0 to 40%, 0 to 30%, 0 to 20% or 0 to 10% of the corresponding (R)-isomer. More preferably, the (S)-isomer is in a form containing 0 to 5% of the corresponding (R)-isomer. Most preferably, the (S)-isomer is in a form containing 0% or no detectable amount of the corresponding (R)-isomer. All percentages hereinbefore are percentages of the mixture by weight. The presence of (R)-isomer may, for example, be routinely detected by the comparison of the optical rotation of a sample of the isomeric mixture with that of a pure sample of the (S)-isomer, or by the $^1$H nmr spectrum of a sample of the isomeric mixture in the presence of a chiral shift reagent or chiral solvating agent.

The invention provides a process for the preparation of the (S)-isomer of a compound of formula (I) or a derivative thereof as defined which process comprises reducing a compound of formula (II):

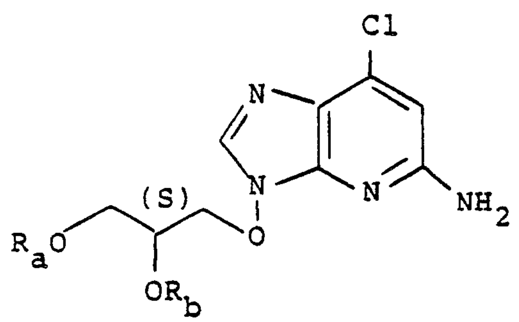

(II)

wherein $R_a$ and $R_b$ are hydrogen or protecting groups, and thereafter optionally converting $R_a$ and/or $R_b$ to OH or forming a derivative thereof as defined in formula (I) and/or forming a pharmaceutically acceptable salt thereof.

The reduction may preferably be achieved using catalytic methods, such as palladium or charcoal in an inert solvent, such as methanol or ethanol, at reflux temperatures. The hydrogen source may cyclohexene or, more preferably, ammonium formate.

One suitable protecting group is that wherein $R_a$ and $R_b$ are joined, forming a 1,3-dioxolan ring, prepared by reaching the corresponding compound wherein $R_a$ and $R_b$ are H, with 2,2-dimethoxypropane. This group, may if desired, be removed by acidic hydrolysis. It will be appreciated that compounds of formula (I) wherein $R_a$ and $R_b$ form a 1,3-dioxolan ring are also active compounds within the ambit of formula (I).

Pharmaceutically acceptable salts, O-acyl derivatives and phosphate derivatives may be prepared conventionally, for example as described in EP-A-141927 and EP-A-182024.

Acyl derivatives of compounds of formula (I) may be prepared by acylating an optionally protected compound of formula (I) in accordance with conventional acylating processes known in the art, and where necessary, deprotecting the resulting product.

The acylation reaction may be carried out by using an acylating agent containing a suitable carboxylic acid acyl group.

Examples of acylating agents suitable for the above process are carboxylic acids, acid halides, such as chlorides or acid anhydrides, preferably anhydrides or acids.

When the acylating agent is a carboxylic acid, a condensation promoting agent such as dicyclohexylcarbodiimide should be included, but this is not necessary when the acylating agent is an acid anhydride.

The acylation reaction may produce a single acyl derivative of a compound of formula (I), or a mixture of derivatives, depending on a number of factors, such as the relative amounts and chemical natures of reactants, the physical conditions of the reaction, and the solvent system. Any mixture produced in this way may be separated into its pure components using standard chromatographic techniques.

The above described acylation process of the invention can yield mono- or di-acylated derivatives of compounds of formula (I), according to the form of protection/deprotection utilised. The following are examples of products obtained by different methods:

(a) Acylated derivatives of the OH groups when both acyl groups are the same, may be obtained by direct acylation of compounds of formula (I).

(b) Mono-acylated derivatives of one OH group may be obtained by acylation of protected intermediates of compounds of formula (I) in which the other -OH group preferably protected by, for example, a monomethoxytrityl or trityl group, and subsequent deprotection by acid treatment. Di-acylated derivatives wherein the acyl groups are different may then be prepared as in (a).

Acyl derivatives of the compounds of formula (I) can be converted to a compound of formula (I) by conventional deacylation or partial deacylation processes. For example, reaction with methanolic ammonia can be used to effect complete deacylation to yield a compound of formula (I) both OH groups are deacylated. Reaction with a mild base such as potassium carbonate can result in partial deacylation of a di-acylated derivative to produce a compound of formula (I) wherein one acyl group and one OH group are present.

Phosphate derivatives are formed by reaction with a phosphorylating agent such as phosphorus oxychloride in pyridine. The $NH_2$ and an OH group are protected as desired or necessary, preferably using a trityl or methoxytrityl protecting group, removable by acid hydrolysis, using acetic acid.

When both OH groups are phosphorylated, a cyclic phosphate derivative is produced with phosphorus oxychloride

Another suitable phosphorylating agent is cyanoethyl phosphoric acid, in which case the product is normally treated with aqueous ammonia, which yields the ammonium salt of the phosphate ester as the final product.

A monophosphate may be converted to a cyclic phosphate using a dehydrating agent, such as dicyclohexylcarbodiimide.

Cyclic acetal derivatives of the compounds of formula (I) may be prepared from the compound of formula (I), using an acyclic acetal, such as $R_{10}O\text{-}C(C_{1-3}alkyl)_2\ \text{-}OR_{10}$ wherein $R_{10}$ is $C_{1-4}$ alkyl, such as methyl or ethyl. The reaction is preferably carried out in an inert solvent such as tetrahydrofuran or dimethylformamide in the presence of an acid such as p-toluenesulphonic acid.

Cyclic carbonate derivatives or the compounds of formula (I) may be prepared from the compound of formula (I), wherein the $-NH_2$ group is preferably protected; with phosgene or 1,1-carbonyldimidazole, and thereafter deprotecting where necessary. Suitable protecting groups include trityl and monomethoxytrityl. The reaction is preferably carried out in dry pyridine at 0-50 °C, preferably at ambient temperature.

It will be appreciated that deprotections and derivative formations may take place in any desired or necessary order.

Compounds of the formula (II) may be prepared according to the following reaction scheme:

Scheme 1

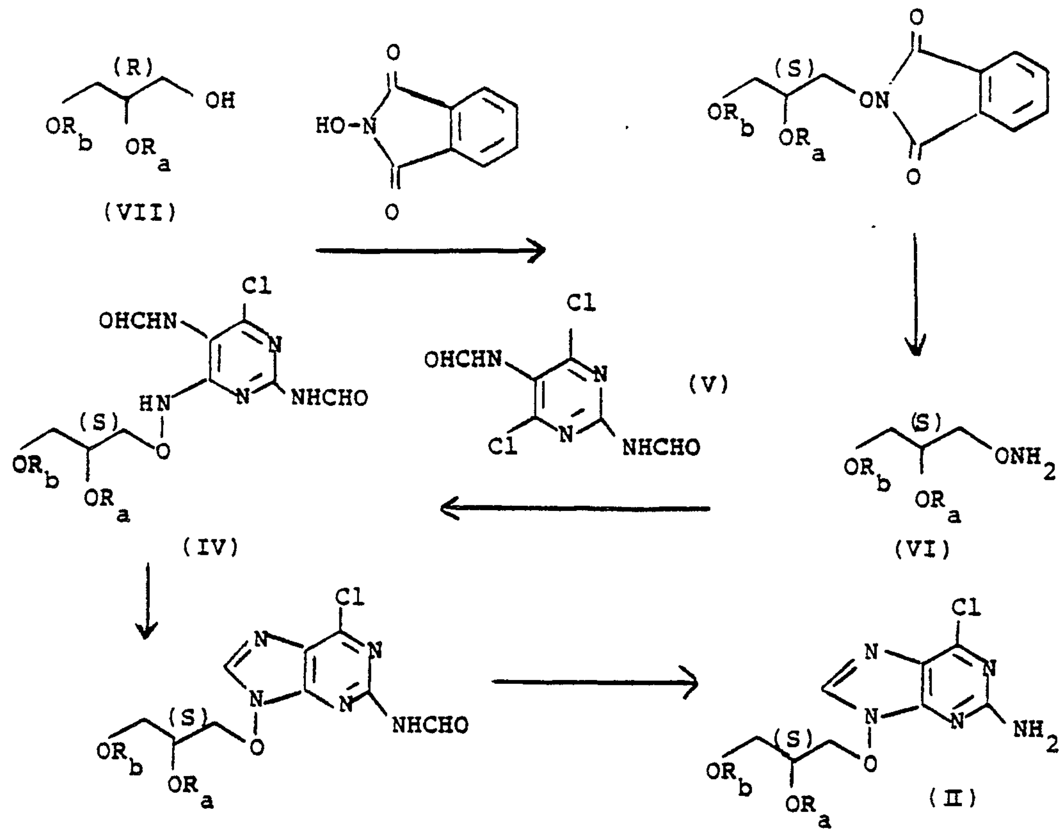

Compounds of the formula (VII) are known or prepared by analogous processes to those used for the preparational structurally similar known compounds. The compound of formula (V) may be prepared by formylation, using formic acid and acetic anhydride, of 2,5-diamino-4,6-dichloropyrimidine, a compound described in J. Org. Chem. 40 (21), 3141, 1975.

Compounds of formulae (II), (III), (IV) and (VI) are novel and form an aspect of the invention.

The present invention also provides a process for the preparation of the (S)-isomer of a compound of formula (I), optionally in admixture with up to 45% of the mixture by weight of the corresponding (R)-isomer, which process comprises resolution of a mixture of (S)- and (R)-isomers of a compound of formula (I) to the extent that the resolution fraction which predominantly contains (S)-isomer optionally contains up to 45% of that resolution fraction by weight of the corresponding (R)-isomer; and optionally forming a derivative as defined, and/or pharmaceutically acceptable salt thereof.

The term 'resolution' is used herein in the conventional practical sense used in the art to include partial resolution, that is, the separation of a mixture of enantiomers of a compound (in any ratio) into two fractions, one of which is enriched in one enantiomer relative to the initial mixture.

Resolution of the mixture of (S)- and (R)- isomers of a compound of formula (I) may be effected conventionally by derivatising the mixture with a chiral derivatising agent, to form a mixture of diastereomers of a derivative of the compound of formula (I). The components of the mixture may then be separated conventionally, for example by fractional crystallisation. Separation may be complete, or partial, such that a given separated fraction comprises up to 45% of the total fraction by weight of the diastereomeric derivative of the (R)-isomer. Subsequently, the diastereomeric derivative or the desired (S)-isomer, optionally in admixture with the diastereomeric derivative of the (R)-isomer is converted to the desired (S)-isomer of the compound of formula (I), optionally in admixture with up to 45% of the (R)-isomer.

The derivatisation for the process of the present invention is preferably effected on a mixture of (S)- and (R)-isomers of a compound of formula (I) by reacting such a mixture of isomers with an optically active reagent suitable for resolving alcohols. Such reagents include optically active acids or acid derivatives, such as acid chlorides, acid anhydrides or isocyanates.

Salts and derivatives may be prepared as hereinbefore described.

A racemic mixture of isomers may be prepared in accordance with scheme 1 using racemic intermediates of formula (VII) or in accordance with the process described in Example 7 of EP-A-242482.

The preferred process for the preparation of a pure (S)-isomer of formula (I) is, however, from chiral intermediates of formula (II).

The compounds of the invention are useful in the treatment of infections caused by viruses, especially herpesviruses such as herpes simplex type 1, herpes simplex type 2; varicella zoster viruses; and also lentiviruses such as visna virus.

Compounds of the invention may be formulated for use in a pharmaceutical composition. Accordingly, in a further aspect of the invention, there is provided a pharmaceutical composition which comprises a compound of the invention together with a pharmaceutically acceptable carrier or excipient.

A composition which may be administered by the oral route to humans may be compounded in the form of a syrup, tablet or capsule. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the composition may be in the form of a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art, or an ointment composition.

Preferably, the composition of this invention is in unit dosage form or in some other form that may be administered in a single dose. A suitable dosage unit might contain from 50 mg to 1 g of active ingredient, for example 100 to 500 mg.

Such doses may be administered 1 to 4 times a day or more usually 2 or 3 times a day. The effective dose of compound will in general be in the range of from 1.0 to 20 mg/kg of body weight per day or more usually 2.0 to 10 mg/kg per day.

No toxicological effects are indicated at the above described dosage levels.

The invention also provides a method of treating viral infections in a human or non-human animal, which comprises administering to the animal an effective, non-toxic amount of a compound of the invention.

The invention also provides a compound of the invention for use as an active therapeutic substance, in particular for the treatment of viral infections.

The compounds of the invention also exhibit a synergistic antiviral effect in conjunction with interferons; and combination products comprising these two components for sequential or concomitant administration, by the same or different routes, are therefore within the ambit of the present invention.

The following example illustrates the invention; the following description illustrate the preparation of intermediates.

## Description - Preparation of Intermediates

### (a) (S)-N-(2,2-Dimethyl-1,3-dioxolan-4-ylmethoxy)phthalimide

A mixture of (R)-2,2-dimethyl-1,3-dioxolane-4-methanol (7.3g, 0.05mol), N-hydroxyphthalimide (8.15g, 0.05mol) and triphenylphosphine (13.11g, 0.05mol) in tetrahydrofuran (200ml) was cooled to 0°C and stirred during the addition of diethyl azodicarboxylate (9.57g, 0.066mol). The dark red solution was stirred at 25°C for 18h when the colour had turned to pale yellow, and the solution was evaporated to dryness. The residue was extracted once with ether (200ml) and the ether removed under reduced pressure. The residue was chromatographed in chloroform-hexane 10:1 affording (S)-N-(2,2-dimethyl-1,3-dioxolan-4-ylmethoxy)-phthalimide (10.5g, 69%) as a white solid; m.p. 99-100°C, $[\alpha]_D^{25}$-14.6° (0.4 in MeOH); $\nu_{max}$ (KBr) $[\alpha]_D^{25}$ 1790, 1730 and 1610cm$^{-1}$; δH($CDCl_3$) 1.35(3H,S,$CH_3$), 1.41(3H,S,$CH_3$), 3.99-(1H,q,J5.5,8.8Hz,$CH_2ON/CH_2OCMe_2$), 4.17(2H,m,1H of $CH_2ON$ + 1H of $CH_2OCMe_2$), 4.32-(1H,q,J5.7,10.1Hz,$CH_2ON/CH_2OCMe_2$), 4.50(1H,m,CH) and 7.75-7.87(4H,m,aromatic). Found: C,60.53; H,5.46; N,5.03%. $C_{14}H_{15}NO_5$ requires C,60.64; H,5.45; N,5.05%.

(b) (S)-2,2-Dimethyl-1,3-dioxolan-4-ylmethoxyamine

A mixture of (S)-N-(2,2-Dimethyl-1,3-dioxolan-4-ylmethoxy)phthalimide (10g, 36mmol), and N-methylhydrazine (3.32g, 72mmol) in dichloromethane (150ml) was stirred at 25° C for 2h. The reaction was filtered, evaporated to dryness and the residue suspended in ether. The suspension was filtered and evaporated and the residue chromatographed in ethyl acetate affording (S)-2,2-Dimethyl-l,3-dioxolan-4-ylmethoxyamine (4.8g, 91%) as a clear liquid $[\alpha]_D^{25}$ -2.4° (0.49 in MeOH); $\nu_{max}$ (film) 3550, 3300 and 1600cm$^{-1}$; δH($CDCl_3$)-1.38(3H,S,$CH_3$), 1.44(3H,S,$CH_3$), 3.71 (3H,m,$CH_2$ON + 1H of $CH_2OCMe_2$), 4.05(1H,q,J6.3,8.2Hz,1H of $CH_2OCMe_2$), 4.35(1H,m,CH) and 5.57(2H,br.s, $D_2O$ exchangeable, $NH_2$); m/z 132($M^+$-$CH_3$, 24%). Found: C,48.76; H,9.00; N,9.56% $C_6H_{13}NO_3$ requires C,48.96; H,8.90; N,9.52%

(c) (S)-4-Chloro-2,5-diformamido-6-(2,2-dimethyl-1,3-dioxolan-4-ylmethoxyamino)pyrimidine

A mixture of 4,6-dichloro-2,5-diformamidopyrimidine (7.3g, 31.1mmol), (S)-2,2-dimethyl-1,3-dioxolan-4-ylmethoxyamine(4.6g, 31.1mmol) and diisopropylethylamine (8.03g, 62.1mmol) in diglyme (125ml) was stirred at 100° C for 4h. The reaction was filtered, evaporated and the residue chromatographed twice in chloroform-methanol 15:1 affording (S)-4-chloro-2,5-diformamido-6-(2,2-dimethyl-1,3-dioxclan-4-ylmethoxyamino)pyrimidine(2.6g, 24%). $\nu_{max}$ ($CHCl_3$) 3400, 1700, 1580, 1560 and 1470cm$^{-1}$; δH($CDCl_3$) 1.39-(3H,S,$CH_3$), 1.48(3H,S,$CH_3$), 3.60-4.65(5H,m,$CH_2$ONH + $CH_2OCMe_2$ + $CHOCMe_2$), 7.96(1H,br.m, $D_2O$ exchangeable, NH), 8.34(1H,S,CHO), 9.25(2H,br.m,$D_2O$ exchangeable, 2XNH) and 9.50(1H,S,CHO); m/z 344-($M^+$-H,<1%), 330($M^+$-$CH_3$,<1%).

(d) (S)-6-Chloro-9-(2,2-dimethyl-1,3-dioxolan-4-ylmethoxy-2-formamidopurine

A solution of (S)-4-chloro-2,5-diformamido-6-(2,2-dimethyl-1,3-dioxolan-4-ylmethoxyamino)pyrimidine (2.1g, 6.08mmol) in diethoxymethyl acetate (30ml) was stirred at 120° C for 3h. The reaction was evaporated to dryness, the residue dissolved in methanol (40ml) and 0.88 ammonia (1ml) and stirred for 1.5h. at 25° C. Evaporation and chromatography of the residue on silica eluting with ethyl acetate-hexane 1:1 gave (S)-6-chloro-9-(2,2-dimethyl-1,3-dioxolan-4-ylmethoxy)-2- formamidopurine (1.3g, 65%) as a white solid; m.p. 147-8° C; $\nu_{max}$ (KBr) 3420, 3200, 1700, 1610, 1580, 1510, and 1440cm$^{-1}$; δH($CDCl_3$) 1.40-(3H,S,$CH_3$), 1.44(3H,S,$CH_3$), 3.89(1H,m,$CH_2OCMe_2$), 4.16(1H,m,$CH_2OCMe_2$), 4.50(3H,m,CH + $CH_2$ON), 8.23-(1H,S,H-8), 8.45(1H,br.d,J11.5Hz,$D_2O$ exchangeable, NH) and 9.59(1H,d,J11.5Hz,HCONH). Found C,43.75; H,4.29; N,21.23%; $M^+$327.0734; $C_{12}H_{14}ClN_5O_4$ requires C,43.98; H,4.31; N,21.37%; $M^+$327.0734.

(e) (S)-2-Amino-6-chloro-9-(2,2-dimethyl-1,3-dioxolan-4-ylmethoxy)purine

A solution of (S)-6-chloro-9-(2,2-dimethyl-1,3-dioxolan-4-ylmethoxy)-2-formamidopurine (400mg, 1.22mmol) in methanol (10ml) and 0.88 ammonia (10ml) was stirred at 25° C for 4h. The solution was evaporated under reduced pressure and the residue chromatographed on silica, eluting with ethyl acetate-hexane 2:1 affording (S)-2-amino-6-chloro-9-(2,2-dimethyl-1,3-dioxolan-4-ylmethoxy)purine (270mg, 74%) as a white solid; m.p. 118-120° C. $\nu_{max}$ (KBr) 3450, 3320, 3210, 1650, !630, 1620, 1560, 1510 and 1470cm$^{-1}$; δH($CDCl_3$) 1.36(3H,S,$CH_3$), 1.42(3H,S,$CH_3$), 3.85(1H,m,$CH_2OCMe_2$), 4.14(1H,m,$CH_2OCMe_2$), 4.43-(3H,m,CH + $CH_2$ON), 5.51(2H,br.s,$D_2O$ exchangeable, $NH_2$), 8.00(1H,S,H-8). Found: C,44.45; H,4.69; N,23.31%; $M^+$299.0795; $C_{11}H_{14}ClN_5O_3$ requires C,44.08; H,4.71; N,23.37%; $M^+$299.0785.

(f) (S)-2-Amino-6-chloro-9-(2,3-dinydroxyprop-1-oxy)purine

A solution of (S)-2-amino-6-chloro-9-(2,2-dimethyl-1,3-dioxolan-4-ylmethoxy)purine (250mg, 0.83mmol) in 80% acetic acid (20ml) was stirred at 25° C for 2h. and then at 70° C for 1h. The solution was evaporated to dryness under reduced pressure, the residue absorbed on silica and chromatographed eluting with acetone-hexane 3:1 affording (S)-2-amino-6-chloro-9-(2,3-dihydroxyprop-1-oxy)purine (170mg, 78%) as a

white solid; m.p. 155-8 °C. $\nu_{max}$ (KBr) 3340, 3200, 1660, 1620, 1560, 1520 and 1470cm$^{-1}$; $\delta$H[$(CD_3)_2SO$] 3.41(2H,m,C$\underline{H_2}$OH) 3.78(1H,m,CH), 4.19(1H,q,J7.4,10.4Hz,$CH_2$ON), 4.40(1H,q,J3.3,10.4Hz,$CH_2$ON), 4.73-(1H,t,J5.0Hz,$D_2O$ exchangeable, OH), 5.14(1H,d,J5.0Hz,$D_2O$ exchangeable, OH), 7.12(2H,S,$D_2O$ exchangeable, $NH_2$) 8.35(1H,S,H-8). Found $M^+$ 259.0475; $C_8H_{10}ClN_5O_3$ requires $M^+$ 259.0472.

Example

(S)-2-Amino-9-(2,3-dihydroxyprop-l-oxy)purine

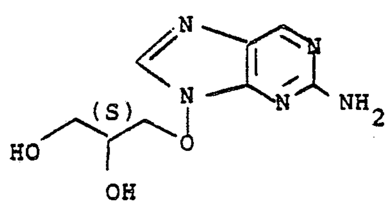

A mixture of (S)-2-amino-6-chloro-9-(2,3-dihydroxyprop-1-oxy)purine(160mg, 0.62mmol), ammonium formate (156mg, 2.5mmol)and 10% palladium on charcoal (20mg) in methanol (5ml) was heated under reflux for 3h. After this time, more ammonium formate (150mg, 2.5mmol) and 10% palladium on charcoal (20mg) were added and the reaction again heated under reflux for 1h. The mixture was filtered hot through a glass fibre paper and the filtrate evaporated under reduced pressure. The residue was recrystallised from hot ethanol affording (S)-2-amino-9-(2,3-dihydroxyprop-1-oxy)purine (100mg, 72%) as a white solid; m.p. 153-5 °C. $[\alpha]_D^{25}$ + 16.5° (0.2 in $H_2O$); $\lambda_{max}$ ($H_2O$) 221 ($\epsilon$25,000), 305($\epsilon$7250)nm; $\nu_{max}$ (KBr) 3370, 3310, 3190, 1650, 1620, 1580, 1520, 1480 and 1440cm$^{-1}$; $\delta$H[$(CD_3)_2SO$) 3.43(2H,m,C$\underline{H_2}$OH), 3.77(1H,m,CH), 4.18-(1H,q,J7.8,9.7Hz,$CH_2$ON), 4.40(1H,q,J2.5,10.7Hz,$CH_2$ON), 4.72(1H,t,J5.4Hz,$D_2O$ exchangeable, OH), 5.16-(1H,d,J4.9Hz,$D_2O$ exchangeable, OH), 6.72(2H,S,$D_2O$ exchangeable, $NH_2$), 8.27(1H,S,H-8), 8.59(1H,S,H-6). Found: C,41.26; H,4.93; N,29.50%; $C_8H_{11}N_5O_3.0.5H_2O$ requires C,41.02; H,5.16; N,29.89%.

Preparation Example

(S)-9-(2,3-Dihydroxyprop-1-oxy)guanine

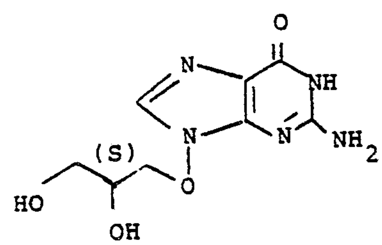

A solution of (S)-6-chloro-9-(2,2-dimethyl-1,3-dioxolan-4-ylmethoxy)-2-formamidopurine (300mg, 0.92mmol) in 80% aqueous formic acid (12ml) was stirred at 100 °C for 2.5h. The solvents were removed under reduced pressure, the residue dissolved in methanol (2ml) and 0.88 ammonia (2ml) and the solution stirred at 25 °C for 2h. The reaction was evaporated to dryness, the residue dissolved in a minimum amount of hot water and filtered through a glass fibre paper. Crystallisation of the filtrate and recrystallisation of the deposited solid from water gave (S)-9-(2,3-dihydroxyprop-1-oxy)guanine (100mg, 45%) as a pale yellow solid; m.p. 252-5 °C dec. $[\alpha]_D^{25}$ + 13.5° (0.16 in $H_2O$); λmax ($H_2O$)252($\epsilon$12,900)nm; νmax (KBr) 3330, 3180, 1690, 1640, 1600 and 1540cm$^{-1}$; $\delta$H[$(CD_3)_2SO$] 3.39(2H,m,C$\underline{H_2}$OH), 3.73(1H,m,$\underline{CH}$OH), 4.10-

(1H,q,J7.7,10.5Hz,$CH_2ON$), 4.32(1H,q,J3.3,10.5Hz,$CH_2ON$), 4.70(1H,t,J5.6Hz,$D_2O$ exchangeable, OH), 5.15-(1H,d,J5.0Hz,$D_2O$ exchangeable, OH), 6.62(2H,br.s,$D_2O$exchangeable, $NH_2$), 7.90(1H,S,H-8), 10.64-(1H,br.s,$D_2O$ exchangeable, NH); $^m/z$ 241($M^+$,13%). Found: C,38.89; H,4.81; N,28.09%; $M^+$241.0820; $C_8H_{11}N_5O_4.0.4H_2O$ requires C,38.68; H,4.79; N,28.19%; $M^+$241.0811.

This compound is believed to be the active metabolite of the compound of the Example.

## Antiviral Activity

### 1. Plaque Reduction Test for Herpes Simplex Viruses 1 and 2.

Cells (Vero or MRC-5) were grown to confluence in 24 well multi-dishes (well diameter = 1.5cm). The drained cell monolayers were each infected with approximately 50 infectious particles of herpes simplex virus 1 (HSV-1; strain HFEM) or herpes simplex virus 2 (HSV-2; strain MS) in 100µl of phosphate-buffered saline. The virus was adsorbed for 1 hour at room temperature. After adsorption, residual inoculum was removed from each well and replaced with 0.5ml of Eagle's MEM containing 5% newborn calf serum and 0.9% agarose (A37). Once the agarose had set, dilutions of the test compound, which had been prepared in Eagle's MEM (containing 5% newborn calf serum), were added, each well receiving 0.5ml of liquid overlay. The test compound was diluted to give the following series of concentrations: 200, 60, 20, 6....0.06µg/ml; final concentrations in the assay ranged, therefore, between 100µg/ml and 0.03µg/ml. The infected cultures were incubated at 37°C in a humidified atmosphere of 5% $CO_2$ until plaques were clearly visible (2 or 3 days for Vero cells, usually 1 day for MRC-5 cells).

Cultures were fixed in formal saline, the agarose overlays were carefully washed off, and then the cell monolayers were stained with carbol fuchsin. A stereo microscope was used to count plaques. The $IC_{50}$ - (concentration to drug which inhibits plaque number by 50% relative to the number of plaques observed in virus control monolayers) of the test compound was calculated. In addition, the monolayers were examined for evidence of drug-induced cytotoxicity; the minimum concentration at which cytotoxicity occurs was recorded.

### 2. CPE Inhibition Test (Established Monolayer) for Lentiviruses

3 x $10^4$ sheep choroid plexus (SCP) cells were plated into individual wells of a 96 well microtitre plate in 100µl of Eagle's MEM with Hanks salts containing 10% heat inactivated foetal calf serum (FCS). When monolayers had become established (after I or 2 days growth) they were washed with 200µl of maintenance medium (Eagle's MEM with Hanks salts containing 0.5% FCS) and infected with 100µl of visna virus (strain K184) in maintenance medium (30 $TCID_{50}$/ml). Test samples were diluted with maintenance medium in further 96 well microtitre plates over the range 200-0.09µg/ml by 3-fold dilution steps. 100µl of the diluted samples was then transferred directly onto virus-infected monolayers (final concentration range therefore 100-0.045µg/ml) and incubated at 37°C in a humidified, 5% $CO_2$ incubation until virus-induced CPE was maximal in the untreated virus-infected controls (usually 12-14 days). The plates were fixed with formal saline and stained with crystal violet. Virus-induced CPE was then scored microscopically and the minimum concentration of sample giving complete protection of the cell monolayers (MIC) determined.

The results were as follows, with (S)-9-(2,3-dihydroxy prop-l-oxy)guanine, the compound of the Preparation Example.

| | $IC_{50}$ |
|---|---|
| HSV-1 (SC16 strain, MRC-5 cells) | 0.7 |
| HSV-2 (MS strain, MRC-5 cells) | 0.06 |
| Visna Virus (K184 strain in SCP cells) | 10 |

This compound was not cytotoxic for uninfected cells at concentrations up to 100 µg/ml.

## Claims

1. The (S)-isomer of a compound of formula (I), optionally in admixture with up to 45% of the mixture by weight of the corresponding (R)-isomer:

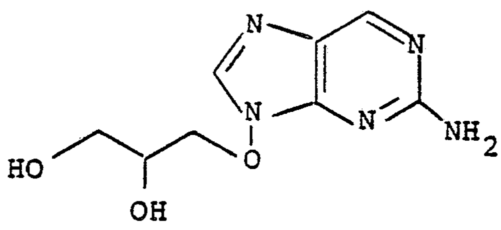

(I)

wherein the OH groups in the chain are optionally in the form of O-acyl, phosphate, cyclic acetal or cyclic carbonate derivatives thereof.

2. A compound according to claim 1 wherein the compound of formula (I) is optionally in the form of a $C_{1-7}$ alkanoyl ester derivative.

3. The (S)-isomer of a compound of formula (I) or a derivative thereof, as defined in claim 1 or 2, in a form containing from 0 to 40% of the corresponding (R)-isomer.

4. The (S)-isomer of a compound of formula (I) or a derivative thereof, as defined in claim 1 or 2, in a form containing from 0 to 5% of the corresponding (R)-isomer.

5. The (S)-isomer according to claim 4 in a form containing 0% or no detectable amount of the corresponding (R)-isomer.

6. A process for the preparation of the (S)-isomer of a compound of formula (I) or a derivative thereof as defined in claim 1, which process comprises reducing a compound of formula (II):

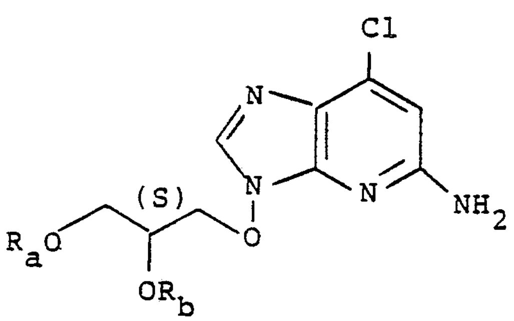

(II)

wherein $R_a$ and $R_b$ are hydrogen or protecting groups, and thereafter optionally converting $R_a$ and/or $R_b$ to OH or forming a derivative thereof as defined in claim 1 and/or forming a pharmaceutically acceptable salt thereof.

7. A compound of formula (VI):

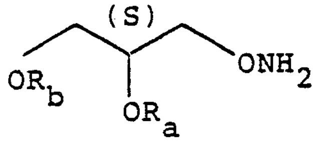

(VI)

wherein $R_a$ and $R_b$ are as defined.

8. A pharmaceutical composition comprising a compound according to any one of claims I to 5, and a pharmaceutically acceptable carrier.

9. A compound according to any one of claims 1 to 5, for use in the treatment of viral infections.

10. Use of a compound according to any one of claims 1 to 5 in the manufacture of a medicament for use in the treatment of viral infections.

Claims for the following Contracting State: ES

1. A process for the preparation of the (S)-isomer of a compound of formula (I), optionally in admixture with up to 45% of the mixture by weight of the corresponding (R)-isomer:

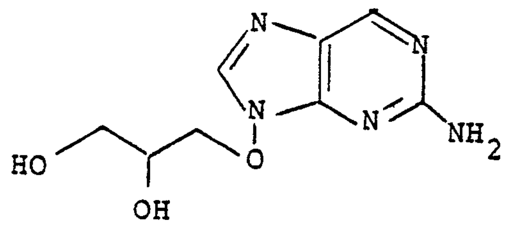

(I)

wherein the OH groups in the chain are optionally in the form of O-acyl, phosphate, cyclic acetal or cyclic carbonate derivatives thereof; which process comprises resolution of a mixture of (S)- and (R)-isomers of a compound of formula (I) to the extent that the resolution fraction which predominantly contains (S)-isomer optionally contains up to 45% of that resolution fraction by weight of the corresponding (R)-isomer; and optionally forming a derivative as defined, and/or pharmaceutically acceptable salt thereof.

2. A process according to claim 1, wherein the resolution fraction contains 0 to 40% of the corresponding (R)-isomer.

3. A process according to claim 1 wherein the resolution fraction contains 0 to 5% of the corresponding (R)-isomer.

4. A process for the preparation of the (S)-isomer of a compound of formula (I) or a derivative thereof as defined in claim 1, which process comprises reducing a compound of formula (II):

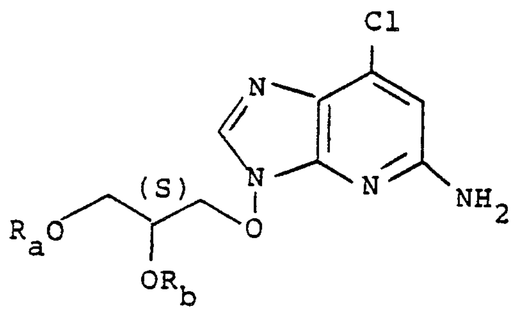

(II)

wherein $R_a$ and $R_b$ are hydrogen or protecting groups, and thereafter optionally converting $R_a$ and/or $R_b$ to OH or forming a derivative thereof as defined in claim 1 and/or forming a pharmaceutically acceptable salt thereof.

5. A process according to claim 4 wherein the compound of formula (I) is optionally in the form of a $C_{1-7}$ alkanoyl ester derivative.

6. Process according to any one of claims 1 to 5 wherein the (S)-isomer is isolated in a form containing 0% or no detectable amount of the corresponding (R)-isomer.

7. Use of an (S)-isomer as defined in claim 1 in the manufacture of a medicament for use in the treatment of viral infections.

8. A method of treatment of viral infections in mammals, which comprises the administration of an effective amount of an (S)-isomer as defined in claim 1.